# EUROPEAN PATENT APPLICATION

(11) **EP 4 814 483 A1**
(43) Date of publication of application: **30.09.2026**
(21) Application number: 24894637.8
(22) Date of filing: 21.11.2024
(51) Int. Cl.: G01N 27/623, G01N 27/626, H01J 49/40

(54) **METHOD FOR IMPROVING SENSITIVITY OF MASS SPECTROMETER USING REFERENCE PROTEIN**

(30) Priority: 21.11.2023 KR 20230162069
(71) Applicant: Seegene Medical Foundation, Seoul 02637 (KR)
(72) Inventor: BAEK, Je Hyun, Gwacheon-si, Gyeonggi-do 13824 (KR); YANG, Won Suk, Bucheon-si, Gyeonggi-do 14774 (KR); CHEON, Dong Huey, Seongnam-si, Gyeonggi-do 13528 (KR); JANG, Heejung, Seoul 02603 (KR); CHOI, Yoon Kyung, Gwangmyeong-si, Gyeonggi-do 14243 (KR)
(74) Representative: Mewburn Ellis LLP
(86) International application number: PCT/KR2024/018491
(87) International publication number: WO 2025/110745

(57) **Abstract**

The present invention relates to a method for optimizing mass spectrometer parameters for the efficient detection of proteins. The present invention enables the establishment of optimal parameters tailored to the target protein by pre-screening for the Pulse Ion Extraction (PIE) value, laser power, and/or detector gain value that maximize the sensitivity and accuracy of the detection signal, using a reference protein with a molecular weight similar to that of the target protein. The present invention enables the detection of trace amounts of the target protein with high sensitivity, accuracy, and reproducibility even when using low-resolution mass spectrometry equipment such as MALDI-TOF, thereby providing highly sensitive analytical results that are not affected by environmental changes at the time of detection. Furthermore, by establishing a linear proportional relationship between the molecular weight of the target protein and the optimal PIE value, and by deriving a linear equation therefrom, the present invention enables simple calculation of an optimal PIE value that maximizes the performance of a mass spectrometer using only the mass information of the target protein, without the need for a reference protein.

## Description

### Technical Field

The present invention relates to a method for significantly improving the analytical reliability and sensitivity of a mass spectrometer, specifically a MALDI-TOF (Matrix-Assisted Laser Desorption/Ionization Time-of-Flight) instrument, by pre-optimizing its key parameters using a reference protein having a molecular weight similar to that of the target protein.

### Background Art

Mass spectrometry is a low-cost, high-efficiency identification system compared to genetic sequence analysis methods such as PCR, and can serve as an important tool for the rapid identification of biomolecules in samples. In particular, protein analysis using a mass spectrometer is suitable for the rapid and accurate detection and identification of microorganisms in samples. A mass spectrometer is fundamentally composed of three parts: an ionization unit, an analyzer unit, and a detection unit, and it is classified according to the structure and operating principles of the ionization and analyzer units employed.

A Matrix Desorption/Ionization Time-of-Flight (MALDI-TOF) mass spectrometer is a device that measures the molecular weight of the target substance by drying a mixture of the sample and a matrix to form a crystalline structure, irradiating it with a laser to desorb and ionize it, and then measuring the time-of-flight (TOF) to the detector. Microbial identification using MALDI-TOF mass spectrometry involves directly analyzing bacterial cells without complex pretreatment, taking only about 5 minutes per species. In particular, since fragmentation does not occur in the target substance, it offers the advantage of rapidly performing mass spectrometry on macromolecules such as proteins, and as a result, it is becoming widely adopted as a microbial identification method in clinical testing. However, MALDI-TOF has limited sensitivity in detecting proteins present at low concentrations. In particular, it suffers from the drawback of result variability caused by analytical environmental factors such as temperature and humidity, during repeated testing of the same sample. Therefore, to enhance sensitivity in the detection of trace proteins, improve repeatability, and increase the reliability of data interpretation, it is important to set the optimal parameters for the MALDI-TOF mass spectrometer to maximize the signal intensity from trace target proteins.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop a reliable mass spectrometry method capable of detecting and identifying the target protein with high sensitivity and reliability, even when it is present in trace amounts in the sample under analysis. As a result, they discovered that, by pre-injecting a reference protein having a molecular weight similar to that of the target protein into the mass spectrometer to identify an optimal pulsed ion extraction (PIE) value at which signal intensity is maximized, full width at half maximum (FWHM) is reduced, and peak-to-valley ratio is increased, and by applying this value as a customized optimal parameter for the target protein, the sensitivity, accuracy, and reproducibility of the analysis can be significantly improved .

Accordingly, it is an object of the present invention to provide a method for optimizing parameters for protein mass spectrometry using a reference protein.

It is another object of the present invention to provide a method for obtaining a pulsed ion extraction (PIE) value based on the mass of the target protein.

Other objects and advantages of the present invention will become more apparent from the following detailed description, the appended claims, and the accompanying drawings.

### Technical Solution

In one aspect of this invention, there is provided a method for optimizing parameters for protein mass spectrometry, comprising:
(a) injecting into a mass spectrometer a reference protein having a mass of 80 to 120% of that of a target protein to be analyzed, or a reference protein having a mass difference of ±5,000 Da or less relative to the target protein; and
(b) selecting a pulsed ion extraction (PIE) value that induces at least one selected from the group consisting of an increase in signal intensity, a decrease in full width at half maximum (FWHM), and an increase in peak-to-valley ratio.

The present inventors have made intensive studies to develop a reliable mass spectrometry method capable of detecting and identifying the target protein with high sensitivity and reliability, even when it is present in trace amounts in the sample under analysis. As a result, they discovered that, by pre-injecting a reference protein having a molecular weight similar to that of the target protein into the mass spectrometer to identify an optimal pulsed ion extraction (PIE) value at which signal intensity is maximized, full width at half maximum (FWHM) is reduced, and peak-to-valley ratio is increased, and by applying this value as a customized optimal parameter for the target protein, the sensitivity, accuracy, and reproducibility of the analysis can be significantly improved .

As used herein, the term "protein" refers to a linear molecule formed by amino acid residues linked together by peptide bonds. In the present invention, the proteins to be detected by mass spectrometry may be proteins that are biological markers, for example, for the presence of a pathogenic strain in a sample (i.e., diagnosis of pathogen infection) or identification of its species and phenotype, or it may be a protein in blood, such as hemoglobin, antibodies, or M proteins.

As used herein, the term "pathogenic strain" refers to any bacteria that act as the cause of an infection or disease, including, for example, but not limited to, *Staphylococcus aureus*, *Streptococcus*, *Escherichia coli*, *Klebsiella pneumoniae*, *Pseudomonas aeruginosa*, *Pseudomonas otitidis*, *Micrococcus luteus*, *Citrobacter koseri*, *Proteus mirabilis*, and *Mycobacterium ulcerans*.

The target proteins to be analyzed in the present invention include, for example, marker proteins that can predict the presence of a pathogenic strain as well as the phenotype thereof, such as antibiotic resistance.

As used herein, the expression "having resistance to antibiotics" means that a specific pathogenic microorganism can grow even in an environment in which antibiotics against the microorganism are present at high concentration or in an effective amount. Whether the pathogenic microorganism has antibiotic resistance may be determined by detecting the presence of an enzyme protein, which is secreted by the pathogenic microorganism and removes or reduces the activities of the antibiotics by degrading the antibiotics. For example, beta-lactam antibiotics that inhibit bacterial cell wall synthesis, such as penicillin, cephalosporin, monobactam, and carbapenem, are inactivated by β-lactamase so that they cannot inhibit pathogens expressing β-lactamase. Accordingly, the term "resistance" is used interchangeably with the term "low therapeutic responsiveness".

As used herein, the term "mass spectrometry" refers to a series of analytical techniques for determining the mass and structural information of any particles, for example, neutral atoms, molecules, clusters (aggregates of atoms or molecules), or polymers. Typically, analyte molecules are impacted with a high-energy beam to form ions, and the generated ions are accelerated through a magnetic field, electrostatic field, or electrodynamic field and separated according to their mass-to-charge ratio (m/z). An ion detector within the mass spectrometer then measures the relative abundance of each ion to generate a spectrum of signal intensity versus m/z.

As used herein, the term "Reference Protein" refers to a standard protein with a known mass value that has a mass similar to that of the target protein to be detected and exhibits detection characteristics such as signal intensity, full width at half maximum (FWHM), and peak-to-valley ratio similar to those of the target protein under the same mass spectrometry conditions.

As used herein, the term "signal intensity" refers to the intensity (i.e., height) of the peak corresponding to the mass value of the target protein. Signal intensity is proportional to the amount of the target protein in the sample and thus provides quantitative information about the target protein. Since the peaks for trace proteins in the sample may be weak and difficult to detect, increasing the signal intensity is very important for the accurate detection and identification of trace proteins.

As used herein, the term "full width at half maximum (FWHM)" refers to a parameter representing the width of a given function, and means the width of the function measured at half of its maximum amplitude. More specifically, it is defined as the difference between two values of the independent variable at which the function reaches half of its maximum value. A lower FWHM value indicates that the peak is narrower and has a more distinct apex, thereby providing a clearer signal indicative of the mass value.

As used herein, the term "peak-to-valley ratio" refers to an index representing the difference between the highest peak and the lowest valley in a mass spectrum, and specifically, it denotes a quantitative value obtained by dividing the difference between the baseline and the peak value by the difference between the baseline and the valley value. The peak-to-valley ratio serves as a measure of how well a specific peak indicating the mass value (m/z) of the target protein is distinguished from peaks indicating other substances.

As used herein, the term "pulsed ion extraction (PIE)" refers to a method in which ion extraction is delayed to compensate for the initial velocity spread of ions generated in a mass spectrometer and to improve mass resolution, thereby ensuring that all ions with the same m/z value are concentrated in time and space along the flight (z) axis within the detector plane. Accordingly, the term "PIE value" refers to the time by which ion extraction from the ion source to the flight tube is delayed. The PIE value is typically measured in nanoseconds (ns), and the optimal PIE value for each target protein is a critical parameter for improving mass accuracy and resolution.

As used herein, the term "laser power" refers to the intensity of the laser energy irradiated to ionize the target protein in mass spectrometry.

As used herein, the term "detector gain" refers to the parameter used to measure and amplify the generated ion signal by the detector in mass spectrometry.

According to the present invention, by pre-injecting a reference protein having a mass in a similar range to the target protein into a mass spectrometer and varying the PIE value, a key parameter that determines the accuracy of the analysis, the method of the present invention may maximize the efficiency of mass spectrometry through searching for an optimal PIE tailored to the target protein which increases signal intensity and the peak-to-valley ratio while reducing FWHM.

According to a specific embodiment, the method of the present invention further comprises, after step (b), step (c) of selecting a primary laser power value, a primary detector gain value, or a combination thereof that induces at least one selected from the group consisting of an increase in signal intensity, a decrease in full width at half maximum (FWHM), and an increase in peak-to-valley ratio under the selected PIE value.

According to the present invention, after fixing the optimal PIE value through the aforementioned steps, the present invention can further improve the analytical sensitivity and accuracy for the target protein by additionally performing a step of optimizing the laser energy range and/or detector gain range for ionizing the target protein under the same criteria (increase in signal intensity, decrease in full width at half maximum, and increase in peak-to-valley ratio).

As used herein, the terms "primary laser intensity" and "primary detector gain" refer to the optimal laser intensity value and detector gain, respectively, that were initially selected based on changes in signal intensity, FWHM and peak-to-valley ratio after fixing the optimal PIE value set by the method of the present invention. The primary laser intensity and primary detector gain may themselves serve as the final parameter values for directly analyzing the target protein, or may serve as preliminary values for establishing cut-off values of signal intensity and FWHM, which are used as criteria for resetting optimal laser intensity and detector gain (i.e., setting "secondary laser intensity" and "secondary detector gain") for analyzing the same target protein in a subsequent independent experiment.

According to a more specific embodiment, method of the present invention further comprises, after step (c), step (d) of selecting the signal intensity and the full width at half maximum (FWHM) obtained at the selected primary laser power value, primary detector gain value, or combination thereof as cut-off values for analysis of the target protein.

According to a more specific embodiment, method of the present invention further comprises, after step (d), step (e) of selecting a secondary laser power value, a secondary detector gain value, or a combination thereof that satisfies the selected cut-off values, and applying the same to the analysis of the target protein.

According to the present invention, after establishing a primary laser intensity value, the signal intensity and FWHM obtained at the established primary laser intensity/detector gain can be used as cut-off values in subsequent independent experiments for the same target protein. Laser intensity and detector gain values that satisfy these cut-off values may then be selected as the actual parameters to be applied in the subsequent experiments (i.e., "secondary laser intensity" and "secondary detector gain"). In this manner, the method of the present invention effectively overcomes the drawback of variability in mass spectrometry results caused by analytical environmental factors, such as temperature and humidity, during repeated analyses, by resetting new laser intensity and detector gain values that satisfy the cut-off criteria for each experiment involving the same target protein.

According to a specific embodiment, the mass spectrometry is selected from the group consisting of matrix-assisted laser desorption/ ionization time-of-flight (MALDI-TOF) mass spectrometry, laser Desorption/Ionization Time-of-Flight (LDI-TOF), surface enhanced laser desorption/ ionization time-of-flight (SELDI-TOF) mass spectrometry and electrospray ionization time-of-flight (ESI-TOF). More specifically, the mass spectrometry is matrix desorption/ ionization time-of-flight (MALDI-TOF) mass spectrometry.

MALDI-TOF mass spectrometry is a method in which a sample supported by a matrix is desorbed and ionized by irradiation with a laser, and then the molecular weights of the generated ions are analyzed by measuring the time (Time-of-Flight) taken for the ions to reach a detector. According to this method, it is possible to quickly and accurately measure the mass of a large biomolecule such as a protein, because fragmentation of the target material does not occur. When the ionized molecule is accelerated by an electric field and the flight time is measured, a mass-to-charge ratio (m/z) is generated, and the molecular weight of the target material may be determined through this m/z value.

According to a specific embodiment, the reference protein has a mass of 90 to 105%, more specifically 95 to 105%, and most specifically 98 to 102% of the mass of the target protein.

According to a specific embodiment, the reference protein has a mass difference of ±4,000 Da or less, more specifically a mass difference of ±3,000 Da or less, and most specifically a mass difference of ±2,000 Da or less relative to the target protein.

In another aspect of this invention, there is provided a method for obtaining a pulsed ion extraction (PIE) value for protein mass spectrometry, comprising substituting the mass of a target protein into Equation 1 below:
$PIE \left(ns\right) = AX + B$
wherein A is a coefficient of 0.010 to 0.011, B is a constant of 652 to 662, and X is the mass (Da) of the target protein.

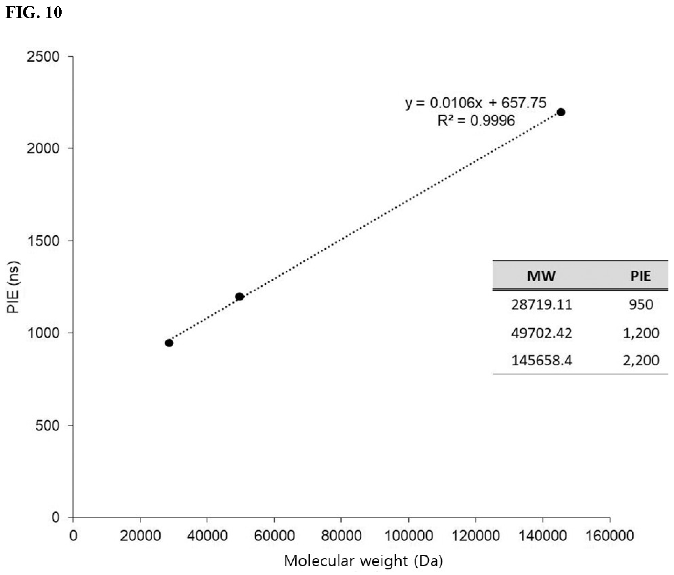

The present inventors investigated the correlation between the molecular weight of a target protein and the optimal PIE value to identify a linear proportional relationship (R² = 0.9996) exists between the two variables (Fig. 10). Accordingly, in addition to the above-described method of the present invention in which a PIE value is determined using a reference protein having a mass similar to that of the analyte protein, an optimal PIE value that maximizes the sensitivity, accuracy, and reproducibility of mass spectrometry can be readily calculated using only the mass information of the analyte protein, even without the use of a reference protein.

According to a specific embodiment, A is a coefficient of 0.0104 to 0.0108 and B is a constant of 657 to 658. More specifically, A is a coefficient of 0.0106 and B is a constant of 657.75.

In still another aspect of this invention, there is provided a composition for performance tuning of a mass spectrometer, comprising as an active ingredient, a reference protein having a mass of 80 to 120% of that of a target protein, or a reference protein having a mass difference of ±5,000 Da or less relative to the target protein.

Since the reference protein and mass spectrometer used in the present invention have already been described above in detail, descriptions thereof are omitted to avoid excessive redundancy.

In the present invention, the term "mass spectrometer performance" refers to the degree to which a mass spectrometer accurately separates target proteins based on their mass-to-charge ratio (m/z). More specifically, an improvement in mass spectrometer performance means that one or more of the following criteria are met: an increase in the signal intensity of the mass spectrum generated by ionizing the target protein, a decrease in FWHM, and an increase in the peak-to-valley ratio. Through this improvement in mass spectrometer performance, the accurate mass values and structural information of the target protein can be obtained, thereby enabling the presence of the target protein in a biological sample to be determined with high reliability. Therefore, the term "mass spectrometer performance tuning" is used synonymously with "mass spectrometer performance enhancement" or "mass spectrometer performance optimization."

As used herein, the term "biological sample" refers to any material likely to contain the target protein to be analyzed or cells, microorganisms, or their culture media that express said protein, including samples isolated from living organisms (e.g., blood, plasma, serum, saliva, tissues, organs, etc.), materials taken from the environment (e.g., water, air, soil, etc.), or artificially mixed samples.

### Advantageous Effects

The features and advantages of the present invention are summarized as follows:
(a) The present invention provides a method for optimizing mass spectrometer parameters for the efficient detection of proteins.
(b) The present invention enables the establishment of optimal parameters tailored to the target protein by pre-screening for the Pulse Ion Extraction (PIE) value, laser power, and/or detector gain value that maximize the sensitivity and accuracy of the detection signal, using a reference protein with a molecular weight similar to that of the target protein.
(c) The present invention enables the detection of trace amounts of the target protein with high sensitivity, accuracy, and reproducibility even when using low-resolution mass spectrometry equipment such as MALDI-TOF, thereby providing highly sensitive analytical results that are not affected by environmental changes at the time of detection.
(d) Furthermore, by establishing a linear proportional relationship between the molecular weight of the target protein and the optimal PIE value, and by deriving a linear equation therefrom, the present invention enables simple calculation of an optimal PIE value that maximizes the performance of a mass spectrometer using only the mass information of the target protein, without the need for a reference protein.

### Brief Description of Drawings

FIG. 1 shows the measurement for signal intensity and full width at half maximum (FWHM) as a function of the pulse ion extraction (PIE) value during MALDI-TOF MS analysis of the KPC-2 protein.
FIG. 2 shows the analysis of signal intensity and half-width at half-maximum (FWHM) as a function of laser power, with the PIE value fixed at 1,000 ns, during MALDI-TOF MS analysis for the KPC-2 protein.
FIG. 3 shows the analysis of signal intensity and half-width at half-maximum (HWHM) as a function of the PIE value during MALDI-TOF MS analysis for an antibody heavy chain.
FIG. 4 shows the analysis of signal intensity and half-width at half-maximum (FWHM) as a function of laser power, with the PIE value fixed at 1,200 ns, during MALDI-TOF MS analysis for an antibody heavy chain.
FIG. 5 shows the analysis of signal intensity and half-width at half-maximum (FWHM) as a function of the PIE value during MALDI-TOF MS analysis for the full-length antibody.
FIG. 6 shows the analysis of signal intensity and half-width at half-maximum (HWHM) as a function of laser power after fixing the PIE value at 2,200 ns during MALDI-TOF MS analysis for the full-length antibody.
FIG. 7 shows the analysis of signal intensity and half-width at half-maximum (HWHM) as a function of PIE value during MALDI-TOF MS analysis for a sample consisting of a 1:1 mixture of CTX-M-15 and CTX-M-1 proteins.
FIG. 8 shows the analysis of signal intensity and half-width at half-maximum (FWHM) as a function of laser power during MALDI-TOF MS analysis for a mixed sample of CTX-M-15 and CTX-M-1 proteins, with the PIE value fixed at 900 ns.
FIG. 9 shows the results of MALDI-TOF MS analysis for a mixed sample of CTX-M-15 and CTX-M-1 proteins, in which the detector gain was varied while the laser intensity was fixed at 82.
FIG. 10 shows a linear correlation between the molecular weight of the target protein and the PIE value applied in mass spectrometry.

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Examples

### Example 1: Preparation of PT (Performance Tuning) Samples

### Expression and Purification of E. coli Proteins

*E. coli* transformed with plasmids containing the KPC-2, CTX-M-1 and CTX-M-15 genes was inoculated into Luria-Bertani (LB) liquid medium containing 50 µg/ml of the antibiotic ampicillin and cultured at 37°C for at least 16 hours. For sample pretreatment, the culture supernatant from strains expressing KPC-2, CTX-M-1 and CTX-M-15 proteins was centrifuged at 4,000 RPM for 15 minutes, and the supernatant was removed to harvest the cells. The harvested cells were resuspended in hypertonic lysis buffer (500 mM NaCl, 20 mM Tris-HCl, pH 8.0). The suspension was incubated at room temperature for 10 minutes, and then centrifuged at 14,000g for 10 minutes at 4°C, after which the supernatant was removed. The remaining cells were resuspended in triple-distilled water and allowed to react at room temperature for 10 minutes. The mixture was then centrifuged again at 14,000g for 10 minutes at 4°C, and the supernatant was collected and stored.

The collected supernatant was purified via anion exchange chromatography. The flow-through (the fraction that did not bind to the column) was collected, concentrated using a 10 kDa filter, and washed with water. The purified sample was used as a PT sample for analyzing proteins with a molecular weight of approximately 28 kDa.

### Purification of Antibody Protein

10 µg of antibody was treated with 20 units/µl of PNGaseF to undergo a deglycosylation reaction at 37°C for 4 hours. The deglycosylated sample was used as a PT sample for analyzing proteins with a molecular weight of approximately 150 kDa. The deglycosylated antibody was reduced by treating with 10 mM DTT at 56°C for 30 minutes, followed by alkylation by reacting with 10 mM IAA for 30 minutes in the dark. The heavy chain of the antibody in the sample was used as a PT sample for analyzing proteins with a molecular weight of approximately 50 kDa.

### Example 2: Optimization of MALDI-TOF Parameters Using the PT Sample

Parameters were optimized using a Bruker Biotyper Smart LT MALDI-TOF MS instrument. KPC-2, a deglycosylated antibody [IgG1k (Immunoglobulin G, Subclass 1, k light chain) Merck, NIST8671], and the heavy chain of said deglycosylated antibody were each used as PT samples. Additionally, to assess peak resolution, the protein pair CTX-M-15/CTX-M-1 (*Δm* = 102.08 *m*/*z*) was used as a PT sample for MALDI-TOF parameter optimization. After spotting 1 µL of each sample onto a MALDI plate and allowing it to dry, 1µL of a matrix consisting of 20 mg/mL sinapic acid dissolved in 0.1% TFA/50% ACN was spotted and allowed to dry, followed by MALDI-TOF analysis.

### Optimization of the analysis of the 28 kDa protein

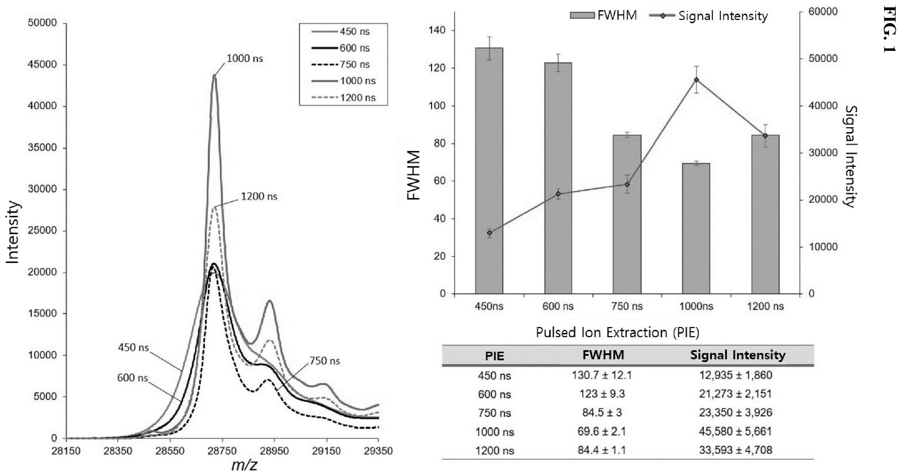

1µL of KPC-2 protein at a concentration of 60 ng/µl was used for the analysis. The KPC-2 protein was tested over a pulsed ion extraction (PIE) range of 450 ns to 1,200 ns (FIG. 1). The peak pattern of the KPC-2 protein revealed that the signal intensity is highest and the full width at half maximum (FWHM) is narrowest at 1,000 ns.

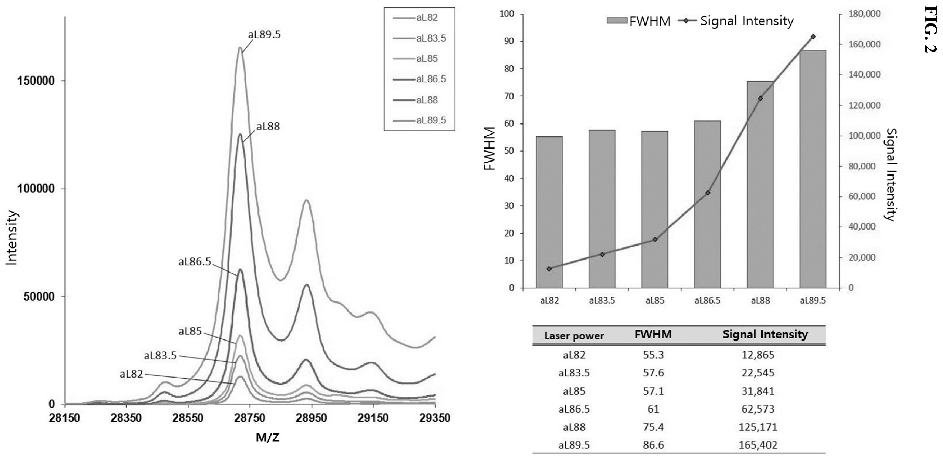

After fixing the PIE value at 1,000 ns, the optimal value was searched by adjusting the laser power from aL82 to aL89.5 (FIG. 2). The peak pattern of the KPC-2 protein showed that as the laser power increases, the signal intensity increases and the full width at half maximum (FWHM) widens. At aL86.5, the FWHM was 61 and the signal intensity was 62,573; from aL88 onward, the FWHM widens sharply and the signal intensity rises rapidly. Accordingly, when the analysis was performed with the laser intensity set to 86.5, cut-off values were established as a full width at half maximum (FWHM) of 65 or less and a signal intensity of 62,000 or more for the KPC-2 peak.

In the case of detector gain, signal intensity increases as the voltage value increases, but this does not significantly affect the FWHM. Therefore, to prevent unnecessary wear on the detector, the actual detector voltage was adjusted within a range of 30 V, and adjustments were made to increase the signal intensity while maintaining the FWHM. In this experiment, the detector voltage was 2,963 V, and a detector gain of 2,967 V was used.

Subsequently, in analyzing molecules with a molecular weight around 28 kDa, 1 µl of KPC-2 protein at a concentration of 60 ng/µl is used. The laser power and detector gain are adjusted to ensure the sample passes the aforementioned cutoff value, and the analysis is then performed.

### Optimization of 50 kDa Protein Analysis

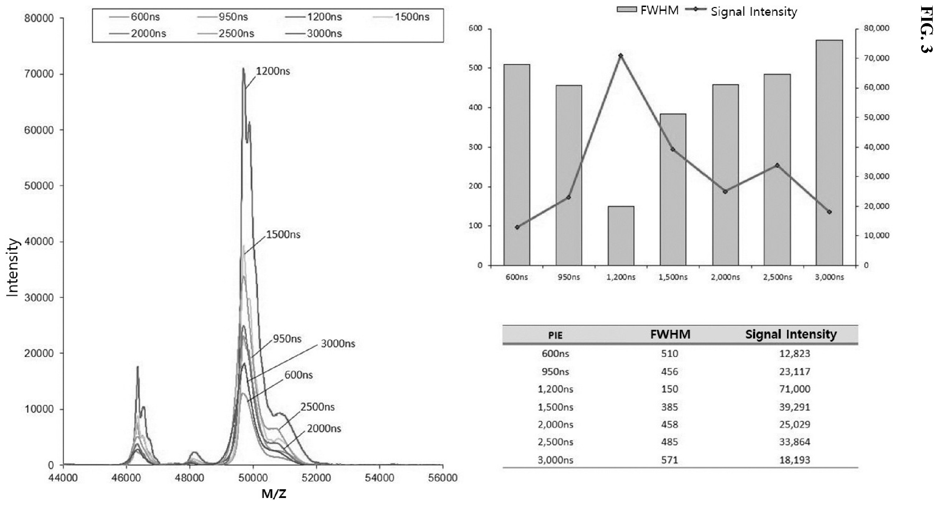

1 µl of antibody heavy chain sample at a concentration of 76 µg/µl was used in the experiment. Testing the pulsed ion extraction (PIE) range from 600 ns to 3,000 ns for the heavy chain sample revealed that at 1,200 ns, the signal intensity in the peak pattern was the highest while the half-width at half-maximum was the narrowest (FIG. 3).

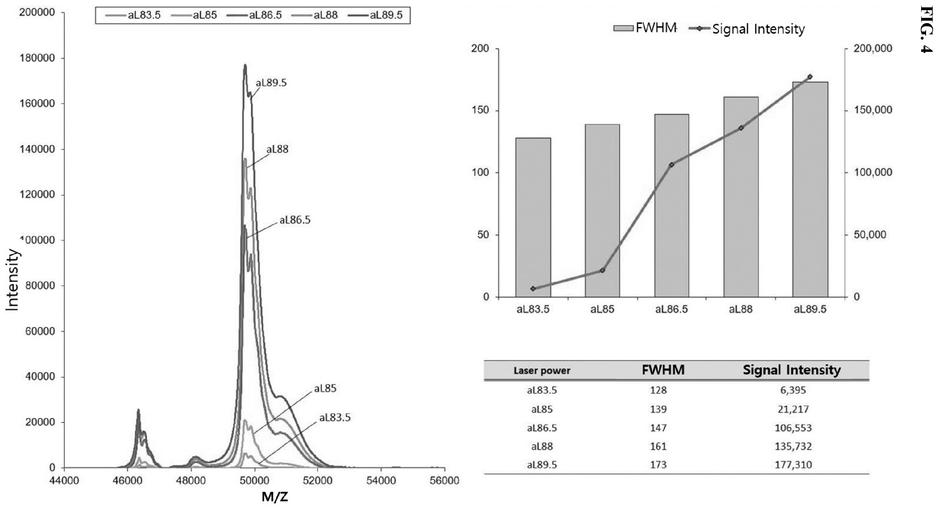

After fixing the PIE value at 1,200 ns, the optimal value was searched by adjusting the laser intensity from aL83.5 to aL89.5. As shown in FIG.4, the peak pattern of the heavy chain protein showed an increase in signal intensity and a widening of FWHM as the laser intensity increased. At aL86.5, the FWHM was 147 and the signal intensity was 106,553, and from aL88 onward, the increase in signal intensity was not significant. Therefore, when analyzing at aL86.5, a FWHM of 150 or less and a signal intensity of 106,000 or more were set as the cutoff criteria for the heavy-chain peak.

In the case of detector gain, although signal intensity increased as the voltage value increased, it did not significantly affect the half-width. Therefore, to prevent unnecessary detector wear, the detector voltage was adjusted within a range of ±30 V from the actual detector voltage. By adjusting the voltage to increase signal intensity while maintaining the FWHM, the detector voltage used in this experiment was set to 2,963 V, and the detector gain was set to 2,967 V.

Subsequently, in analyzing molecules with a molecular weight around 50 kDa, 1 µL of an antibody heavy chain sample at a concentration of 76 µg/µL was used. The laser power and detector gain were adjusted to meet the aforementioned cutoff criteria before analysis.

### Optimization of 150 kDa Protein Analysis

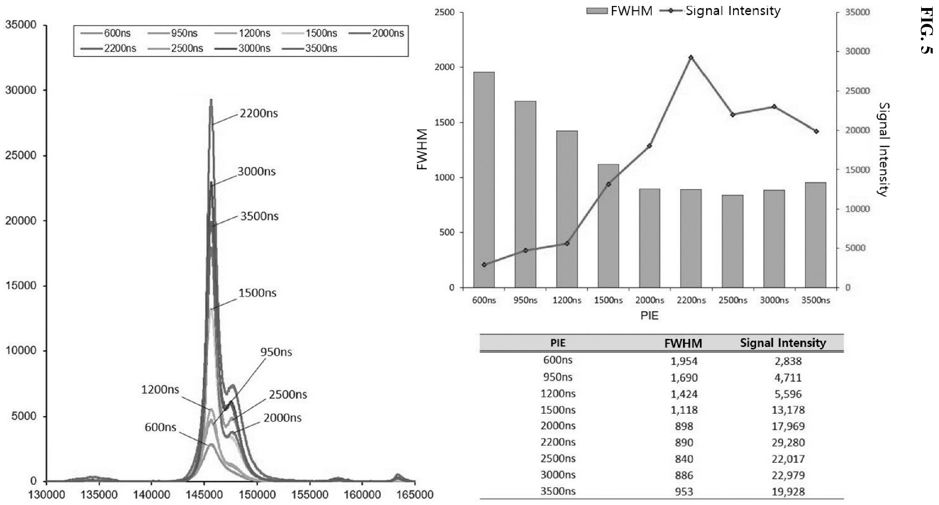

1 µl of an antibody sample at a concentration of 86 ng/µl was used in the experiment. Testing the pulsed ion extraction (PIE) range from 600 ns to 3,500 ns for the antibody sample revealed that the peak pattern of the antibody protein exhibited the highest signal intensity and the narrowest FWHM at 2,200 ns (FIG. 5). Although the FWHM at 2,200 ns was not the lowest value, 2,200 ns was selected as the optimal PIE value considering the increase in signal intensity.

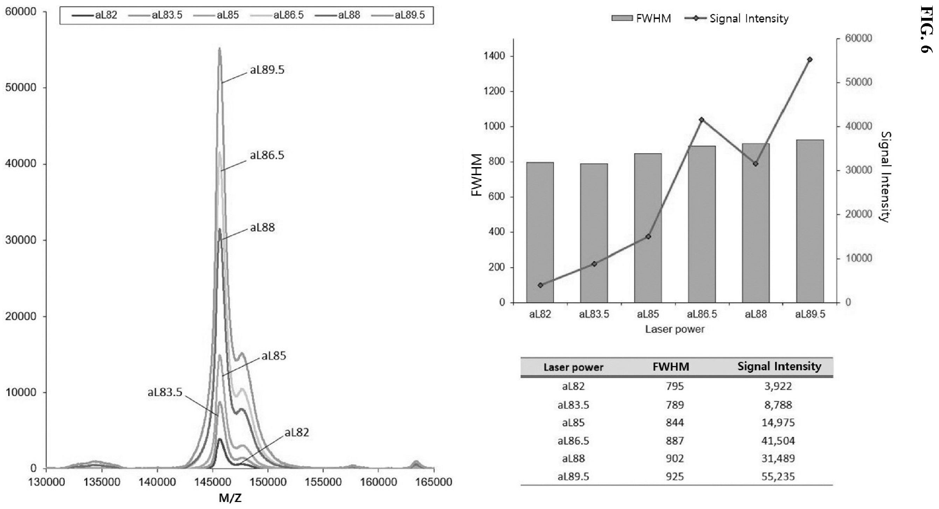

After fixing the PIE value at 2,200 ns, the optimal value was searched by adjusting the laser intensity from aL82 to aL89.5. The peak pattern of the antibody protein showed that as the laser intensity increased, the signal intensity increased and FWHM broadened (FIG. 6). At aL86.5, the FWHM was 887 and the signal intensity was 41,504, and a decrease in signal intensity was observed starting from aL88. Therefore, when analyzing at aL86.5, a FWHM of 890 or less and a signal intensity of 40,000 or more were set as the cutoff criteria for the antibody peak.

Regarding detector gain, while signal intensity increased as the voltage value increased, it did not significantly affect the half-width. Therefore, to prevent unnecessary detector wear, the actual detector voltage was adjusted within the 30 V range. By adjusting the voltage to increase signal intensity while maintaining the FWHM, the detector voltage used in this experiment was set to 2,963 V, and the detector gain was set to 2,967 V.

Subsequently, in analyzing molecular weights around 150 kDa, 1 µl of antibody sample at a concentration of 86 ng/µl was used. The laser power and detector gain were adjusted to meet the aforementioned cutoff criteria before analysis.

### Optimization for distinguishing peaks of similar proteins

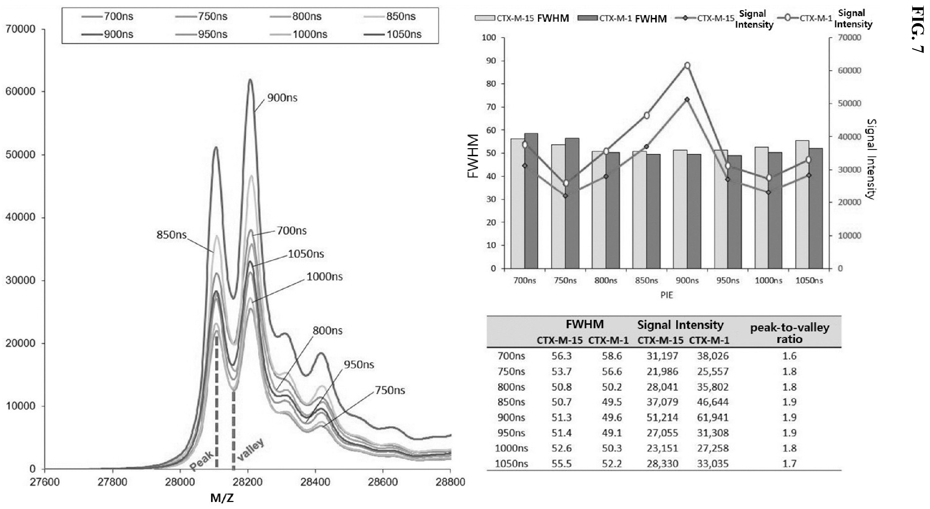

The CTX-M-15 and CTX-M-1 proteins, which have a mass difference of 102 m/z, were mixed in a 1:1 ratio to a concentration of 40 ng/µl. 1µl of sample was then taken and tested within the PIE range of 700 ns to 1050 ns. The results showed that the signal intensity of CTX-M-15 and CTX-M-1 was highest at PIE 900 ns, with narrow FWHM and high peak-to-valley ratio (FIG. 7). Therefore, PIE 900 ns was selected as the optimal value.

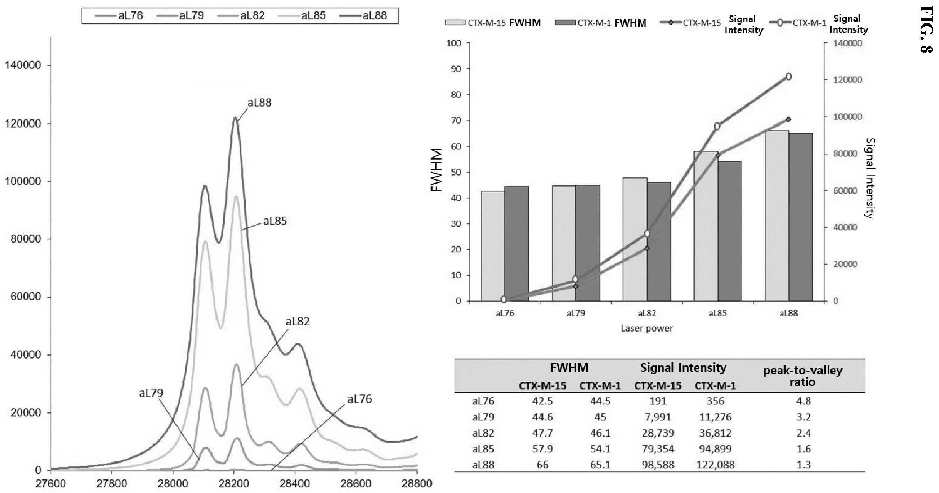

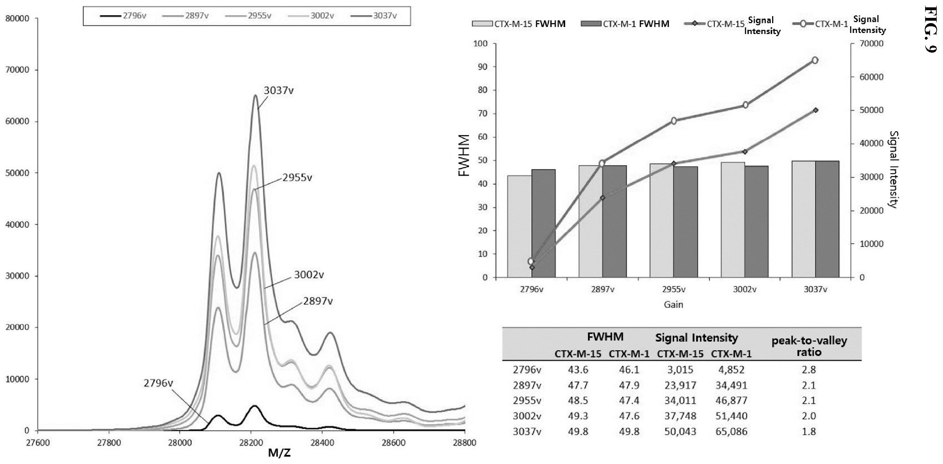

After fixing the PIE value at 900 ns, tests were conducted to find the optimal value by adjusting the laser intensity from aL76 to aL88. As a result, the peak patterns of the CTX-M-15 and CTX-M-1 proteins revealed that as the laser intensity increased, the signal intensity increased and FWHM widened. At aL82, the signal intensity of CTX-M-15 was confirmed to be 28,000 or higher, that of CTX-M-1 was 36,000 or higher, the FWHM for both CTX-M-15 and CTX-M-1 was 50 or lower, and the peak-to-valley ratio was 2 or higher (FIG. 8). After setting the laser power to 82, the detector gain was adjusted. As a result, it was observed that while signal intensity increased as the detector gain increased, this did not significantly affect FWHM (FIG. 9). Therefore, to prevent unnecessary wear on the detector, the detector voltage was adjusted within a range of ±30 V from the actual detector voltage. By adjusting the voltage to increase signal intensity while maintaining the FWHM, the detector voltage in this experiment was set to 2,963 V. At a detector gain of 2,955 V, the signal intensity of CTX-M-15 was 34,000 or higher, and that of CTX-M-1 was 46,000 or higher; FWHM for both CTX-M-15 and CTX-M-1 was 50 or less; and the peak-to-valley ratio was 2 or higher. These values were used as cutoff criteria for distinguishing peaks of similar proteins.

Subsequently, in analyzing proteins with similar molecular weights around 28 kDa, the CTX-M-15 and CTX-M-1 proteins were mixed in a 1:1 ratio to a concentration of 40 ng/µl. Then, using 1 µl of the sample, the laser power and detector gain were adjusted so that the sample would pass the aforementioned cutoff criteria, and analysis was performed.

A quantitative correlation was confirmed between the molecular weight of the target protein and the PIE value applied in mass spectrometry (R²= 0.9996) (FIG. 10).

Having described specific embodiment of the present invention in detail above, it is to be understood that variants and modifications thereof falling within the spirit of the invention may become apparent to those skilled in this art, and the scope of this invention is to be determined by appended claims and their equivalents.

## Claims

1. A method for optimizing parameters for protein mass spectrometry, comprising:
(a) injecting into a mass spectrometer a reference protein having a mass of 80 to 120% of that of a target protein to be analyzed, or a reference protein having a mass difference of ±5,000 Da or less relative to the target protein; and
(b) selecting a pulsed ion extraction (PIE) value that induces at least one selected from the group consisting of an increase in signal intensity, a decrease in full width at half maximum (FWHM), and an increase in peak-to-valley ratio.

2. The method of claim 1, wherein the method further comprises, after step (b), step (c) of selecting a primary laser power value, a primary detector gain value, or a combination thereof that induces at least one selected from the group consisting of an increase in signal intensity, a decrease in full width at half maximum (FWHM), and an increase in peak-to-valley ratio under the selected PIE value.

3. The method of claim 2, wherein the method further comprises, after step (c), step (d) of selecting the signal intensity and the full width at half maximum (FWHM) obtained at the selected primary laser power value, primary detector gain value, or combination thereof as cut-off values for analysis of the target protein.

4. The method of claim 2, wherein the method further comprises, after step (d), step (e) of selecting a secondary laser power value, a secondary detector gain value, or a combination thereof that satisfies the selected cut-off values, and applying the same to the analysis of the target protein.

5. The method of claim 1, wherein the mass spectrometry is selected from the group consisting of matrix-assisted laser desorption/ ionization time-of-flight (MALDI-TOF) mass spectrometry, laser Desorption/Ionization Time-of-Flight (LDI-TOF), surface enhanced laser desorption/ ionization time-of-flight (SELDI-TOF) mass spectrometry and electrospray ionization time-of-flight (ESI-TOF).

6. The method of claim 5, wherein the mass spectrometry is a MALDI-TOF.

7. The method of claim 1, wherein the reference protein has a mass of 95 to 105% of the mass of the target protein.

8. A method for obtaining a pulsed ion extraction (PIE) value for protein mass spectrometry, comprising substituting the mass of a target protein into Equation 1 below: $PIE \left(ns\right) = AX + B$ wherein A is a coefficient of 0.010 to 0.011, B is a constant of 652 to 662, and X is the mass (Da) of the target protein.

9. The method according of claim 8, wherein A is a coefficient of 0.0104 to 0.0108, and B is a constant of 657 to 658.

10. A composition for performance tuning of a mass spectrometer, comprising as an active ingredient, a reference protein having a mass of 80 to 120% of that of a target protein, or a reference protein having a mass difference of ±5,000 Da or less relative to the target protein.

11. The composition of claim 10, wherein the mass spectrometry is selected from the group consisting of matrix-assisted laser desorption/ ionization time-of-flight (MALDI-TOF) mass spectrometry, laser Desorption/Ionization Time-of-Flight (LDI-TOF), surface enhanced laser desorption/ ionization time-of-flight (SELDI-TOF) mass spectrometry and electrospray ionization time-of-flight (ESI-TOF).

12. The composition of claim 10, wherein the reference protein has a mass of 95 to 105% of that of the target protein.
